# EUROPÄISCHE PATENTSCHRIFT

(11) **EP 0 962 443 B1**
(45) Veröffentlichungstag und Bekanntmachung des Hinweises auf die Patenterteilung: **23.04.2003**
(21) Anmeldenummer: 98110086.0
(22) Anmeldetag: 03.06.1998
(51) Int. Cl.: C07C 51/42, C07C 59/265, A61K 9/46, A23L 2/40

(54) **Mit Alkali- bzw. Erdalkali-Ionen dotierte Zitronensäure**
Citric acid enriched in alkali or alkaline-earth ions
Acide citrique enrichi en ions alcalins ou alcalino-terraux

(43) Veröffentlichungstag der Anmeldung: 08.12.1999
(73) Patentinhaber: Dr. Gergely & Co., 1053 Wien (AT)
(72) Erfinder: Gergely, Gerhard, Dr., 1053 Wien (AT); Gergely, Irmgard, 1053 Wien (AT); Gergely, Thomas, Dr., 1053 Wien (AT); Gergely, Stefan, Dr., 1053 Wien (AT)
(74) Vertreter: Büchel, Kurt F.

(56) Entgegenhaltungen:
- EP-A- 0 076 340
- EP-A- 0 642 784
- WO-A-88/00009
- WO-A-93/00886
- CH-A- 662 926

## Beschreibung

Die Erfindung betrifft eine an der Kristalloberfläche mit Alkali- oder Erdalkali-Ionen dotierte Zitronensäure mit verminderter Reaktivität gegenüber CO₂-abspaltenden Bestandteilen, sowie ein Verfahren zu ihrer Herstellung.

Bei der Herstellung von Brause- bzw. Löstabletten bilden eßbare, organische Säuren den Säurebestandteil dieser Tabletten. Für die Herstellung von Brause- bzw. Löstabletten gibt es grundsätzlich zwei Verfahren, und zwar einerseits eine Trockenmischung der Säure und der Alkali- bzw. Erdalkali-Bestandteile sowie andererseits eine Granulierung der Zitronensäure mit den CO₂-abspaltenden Bestandteilen. Bei einer Trockenmischung von Zitronensäure mit Erdalkali- bzw. Alkali(hydrogen)carbonaten hat sich gezeigt, daß die Verpreßbarkeit nicht gut ist und die Tabletten häufig zum "Deckeln" (d.h. zum Zerspringen in einen kompakten Bodenteil und einen weniger kompakten "Deckel"teil) neigen, weiters daß diese Brause- oder Löstabletten über keine ausreichende Stabilität verfügen, da mit Spuren von Restfeuchtigkeiten oder bei Einfluß von Luftfeuchtigkeit bereits Reaktionen der Erdalkali- bzw. Alkali(hydrogen)carbonate mit der eßbaren, organischen Säure, z.B. Zitronensäure, entstehen. Diese Feuchtigkeitssensibilität wirkt sich bei Lagerung auch auf die Stabilität der Tablette aus, sodaß es zwischen den carbonatischen und den Säurebestandteilen zu geringfügigen Reaktionen kommt, die entweder zu einer Verlängerung der Auflöszeit oder zu einer Zerstörung des Systems durch eine nachfolgende Kettenreaktion führen.

Beim Granulierverfahren werden die organischen Säuren, wie z.B. Zitronensäure, mit Erdalkali- bzw. Alkali(hydrogen)carbonaten vermischt und mit einem polaren Lösungsmittel bzw. mit Mischungen aus Wasser und/oder Ethanol granuliert, wobei Granulate aus Zitronensäure mit den Erdalkali- bzw. Alkali(hydrogen)carbonaten geformt werden. Dieses Verfahren liefert qualitativ bessere Produkte, ist aber aufgrund des erforderlichen Energie- und Zeitaufwandes kostenintensiver.

In einer Reihe von Patenten wurde bereits im Zuge der HerStellung von Brausegranulaten die Oberflächenbehandlung von Zitronensäure mit verschiedenen Verfahren geschützte die hauptsächlich darauf beruhen, die Oberfläche von Zitronensäurekristallen in das saure Mono-Natriumcitrat umzuwandeln. Dies läßt sich entweder direkt durch Reaktion mit Natriumhydrogencarbonat oder Natriumcarbonat erreichen.

So wurde bereits in der EP Nr. 0 076 340 vorgeschlagen, Zitronensäure mit Erdalkali- bzw. Alkali(hydrogen)carbonaten zur Reaktion zu bringen und so die Oberfläche der Säurekristalle durch ein Alkali- oder Erdalkalisalz zu passivieren und damit reaktionsträge zu machen. Dabei wird die Oberfläche der Säurekristalle teilweise zum entsprechenden Säuresalz, wie z.B. Mononatriumcitrat oder Di-Natriumcitrat, umgesetzt.

Weiters wurde in der Patentschrift EP Nr. 0 642 784 vorgeschlagen, die Umsetzung zwischen Säure und Carbonat zur Bildung von Alkalisalzen an der Oberfläche der Säurekristalle zu umgehen, indem man die Säurekristalle mit einer Lösung von Natriumcitrat überzieht bzw. abdeckt und auf diese Schicht anschließend wiederum Carbonate oder Bicarbonate durch teilweise Reaktion aufbringt.

Es wurde auch in der EP Nr. 0 272 312 angeführt, die Zitronensäure mit einer Citratlösung als Vorreaktionslösung - hergestellt aus Zitronensäure und Kaliumbicarbonat- zu behandeln, um damit eine Passivierung der Zitronensäure zu erreichen, wobei die aufgebrachte Vorreaktionslösung auf der Zitronensäure als Bindemittel und Puffer zwischen der Zitronensäure und dem anschließend zugefügten und zur Reaktion gebrachten Alkalihydrogencarbonat bzw. Erdalkalicarbonat dient.

In der EP-A1-870 624 wurde auch bereits vorgeschlagen, im Zuge der Herstellung von Brausemischungen die Säurekristalle mit Alkalibicarbonat zu mischen und unter begrenzter Feuchtigkeit zu einer Teilreaktion zu bringen oder sie zwecks Stabilisierung von Brausesystemen durch eine Mischung von Dinatriumcitrat und Mononatriumcitrat zu ersetzen. Beides sind jedoch relativ umständliche und teure Lösungen und sind überhaupt nur dann teilweise brauchbar, wenn gleich im Anschluss daran die restlichen Komponenten (z.B. weiteres Carbonat, Füll-, Wirk-, Süssstoffe und dergleichen) zugefügt werden und die Mischung in Sachets abgefüllt oder zu Tabletten verpresst wird.

Der Bedarf an der Verfügbarkeit einer modifizierten, d.h. oberflächenbehandelten, festen, essbaren, organischen Säure als wesentlich feuchtigkeitstabilerer Lager- oder Handelsware für die zahlreichen bekannten Einsatzzwecke, z.B. für den Einsatz im Bereich der Getränkeerzeugung, der Nahrungsmittelherstellung und -zubereitung oder zur Herstellung von chemischen und/oder pharmazeutischen Produkten wurde bislang noch nicht angesprochen. Auch sind die im vorigen Absatz erwähnten, bekannten Verfahren für eine wirtschaftliche Herstellung einer oberflächenpassivierten organischen Säure nicht geeignet.

In der vorliegenden Erfindung ist es nun aber gelungen, die gewünschten Säurekristalle in einem grosstechnisch preiswerten Verfahren herzustellen, indem die Säurekristalle oberflächlich mit Alkali- oder Erdalkaliionen dotiert werden, was im Zuge einer chemischen Reaktion unter Einsatz von Alkali- oder Erdalkalilauge oder von Trinatriumcitratlösungen und in jedem Fall in Abwesenheit jeglicher CO₂-Entwicklung erfolgt. Das nach dem Trocknen auf diese Weise erhaltene Produkt ist gut lagerfähig und gegenüber Feuchtigkeitseinfluss relativ stabil.

Die Passivierung der Säurekristalloberflächen erfolgte bisher großteils entweder über eine Teilreaktion mit den Erdalkali- bzw. Alkali(hydrogen)carbonaten, wobei an der Oberfläche der Säurekristalle im Falle der Zitronensäure Natriumcitrat gebildet wird, oder durch Aufsprühen entsprechender Lösungen von Mono-, Di- und/oder Tri-Citraten auf die Zitronensäurekristalle und anschließende Anheftungsreaktion der CO₂-abspaltenden Alkalien oder Erdalkalien.

Die Erfindung hat sich nun die Aufgabe gestellt, die eßbaren, organischen Säuren oberflächlich so zu behandeln, daß sie die Nachteile des Standes der Technik weitgehend vermeiden und eine gute Verpreßbarkeit aufweisen sowie eine gute Stabilität der daraus hergestellten Tabletten gegenüber Feuchtigkeit bewirken, sodass die Herstellung von Brauseoder Löstabletten in einer Trockenmischung mit den CO₂abspaltenden Bestandteilen mittels Direktverpressung erfolgen kann. Ein weiterer Vorteil der Erfindung ist die großtechnisch einfache Herstellung der erfindungsgemäß oberflächenbehandelten Säure.

Dies gelingt überraschenderweise dadurch, daß man die eßbaren, organischen Säuren mit Alkali- oder Erdalkali-Ionen dotiert, im Falle der Zitronensäure also Zitronensäuremoleküle an der Oberfläche der Kristalle in Citrat umwandelt, wie dies im kennzeichnenden Teil des Anspruches 1 beschrieben ist, sodaß eine gegenüber den CO₂-abspaltenden Bestandteilen einer Brausetablette wie Erdalkali- bzw. Alkali(hydrogen)carbonaten weniger reaktive Zitronensäure entsteht, die in Mischung mit Erdalkali- bzw. Alkali(hydrogen)-carbonaten stabile Brause- bzw. Löstabletten ergibt. Vorteilhafte Ausführungsformen und Weiterbildungen der Erfindung sind in den abhängigen Ansprüchen beschrieben.

Ein weiterer Vorteil dieser behandelten Säuren besteht darin, daß sie eine bessere Verpreßbarkeit durch die Kristallgitterstörung mit Alkali- bzw. Erdalkali-Ionen zeigen und damit die Direktverpressung einer Mischung aus der dotierten und passivierten Zitronensäure mit Erdalkali- bzw. Alkali(hydrogen)carbonaten möglich ist.

Ein weiteres Ziel der Erfindung ist es, solche Brausesysteme bereitzustellen und ein in der Großtechnik einsetzbares Verfahren zu präsentieren, welches deren Herstellung auf einfache Weise erlaubt. Das Prinzip der erfindungsgemässen Oberflächenmodifikation beruht darauf, daß die Dotierung der Säure mit Alkali- oder Erdalkali-Ionen an der Kristalloberfläche dazu führt, daß die Reaktivität der Zitronensäure gegenüber Erdalkali- bzw. Alkali(hydrogen)carbonaten und anderen Stoffen herabgesetzt wird, indem an der Oberfläche der Zitronensäurekristalle partiell Zitronensäuremoleküle in Natriumcitrate, vorwiegend Mononatriumcitrat und Trinatriumcitrat, umgewandelt werden.

Um eine solche oberflächenmodifizierte organische Säure großtechnisch herzustellen, können verschiedene Wege beschritten werden, welche nachfolgend am Beispiel der Zitronensäure näher beschrieben werden. Es ist daraus für einen Fachmann ohne weiteres ableitbar, daß auch andere eßbare organische Säuren wie z.B. Weinsäure oder Äpfelsäure auf analoge Art und Weise behandelt werden können.

Im Falle der Zitronensäure kann man also beispielsweise die Säurekristalle mit Lösungen aus Alkali- bzw. Erdalkalicitraten oder Alkali- und/oder Erdalkalihydroxyden bzw. mit Ammoniakdampf behandeln, d.h. auf die Zitronensäurekristalle eine Lösung aufbringen und die so behandelten Kristalle anschließend trocknen.

Die großtechnische Zitronensäure-Behandlung kann in einem Mischer erfolgen, in den die Lösungen eingebracht und mit den Säurekristallen vermischt oder auf diese aufgesprüht werden, woran sich ein Trocknungsschritt in einem Tunnel-Trockner oder einer anderen dafür geeigneten Trockenvorrichtung anschliesst. Die Lösung kann aber auch in einem Wirbelschicht-Granulator aufgesprüht und die Feuchtigkeit anschließend weggetrocknet werden. Weiters ist auch ein kontinuierliches Verfahren möglich, bei dem die Zitronensäure mit den Lösungen besprüht und kontinuierlich getrocknet wird. Darüber hinaus besteht auch die Möglichkeit, in einem Vakuumgranulator die Zitronensäure auf ca. 60°C aufzuheizen, die Lösungen einzusaugen, zu verteilen und anschließend mittels Vakuum zu trocknen.

Man kann jedoch die Dotierung mit Alkali- und/oder Erdalkaliionen, beispielsweise Natrium-, Kalium- und/oder Calciumionen, auch bereits bei der Herstellung der Zitronensäure an sich durchführen, indem die Zitronensäurekristalle vor dem Trocknen mit Alkalihydroxyden oder Citratlösungen oder mit Mischungen von Citraten und Alkalihydroxyden behandelt und anschließend getrocknet werden. Durch das Behandeln der Zitronensäure mit Alkalihydroxyden, Citraten oder Mischungen von Alkali- und Erdalkalicitraten mit Alkalihydroxyden erreicht man eine Dotierung der Säurekristalle mit beispielsweise Natrium, Kalium und/oder Calcium und damit eine Passivierung der Oberfläche.

Eine oberflächliche Umsetzung der Zitronensäure, die auch als Monohydrat vorliegen kann, zum Citrat lässt sich auch durch gemeinsames Erhitzen von Zitronensäurekristallen mit wasserhaltigem Trinatriumcitrat erzielen. Es kommt dabei zu einer Reaktion der Zitronensäure mit Trinatriumcitrat, wobei sich an der Oberfläche der Zitronensäure zumindest eine monomolekulare Schicht an Mononatriumcitrat bildet, in Abhängigkeit von der eingesetzten Menge Trinatriumcitrat jedoch auch Dinatriumcitrat- und Trinatriumcitratanteile entstehen können. Die so behandelte Zitronensäure wird anschliessend getrocknet und kann - gegebenenfalls nach Zwischenlagerung - in Trockenmischungen für eine direkt verpreßbare Brausetablette mit CO₂-abspaltenden Bestandteilen, wie Erdalkali- bzw. Alkali(hydrogen)carbonaten Verwendung finden.

Um die Verpreßbarkeit noch zu verbessern, können zu diesen Citrat- oder Hydroxyd-Lösungen, mit denen die Zitronensäure behandelt wird, auch Neutralstoffe wie Zuckeralkohole oder Kolloide zugesetzt werden, die dann beim Trocknen an der Oberfläche der Zitronensäure auskristallisieren bzw. einen Film bilden und damit eine größere Elastizität der Zitronensäure bei der Verpressung gewährleisten. Es können alle wasserlöslichen Zuckeralkohole wie z.B. Mannit, Sorbit, Xylit oder Zucker wie Saccharose und Fructose sowie Hydrokolloide wie Maltodextrin, hydrolisierte Stärke, Gummi arabicum, etc. eingesetzt werden.

Um die gewünschten Effekte zu erzielen, nämlich einerseits eine geringere Reaktivität gegenüber Erdalkali- bzw. Alkali(hydrogen)carbonaten, andererseits eine verbesserte Verpreßbarkeit der essbaren organischen Säure bei der Herstellung von z.B. Brausetabletten zu erzielen, ist eine relativ geringe Dotierung der Säurekristall-Oberflächemit den entsprechenden Ammonium-, Alkali- und/oder Erdalkali-Ionen erforderlich. Die gewünschten Eigenschaften erzielt man schon bei einer Dotierung von 0,01 - 1,5 Gew.% an Ammonium-, Alkali- und/oder Erdalkali-Ionen, bezogen auf die organische Säure, insbesondere Zitronensäure; es ist aber auch möglich, eine Dotierung bis zu 5 Gewichtsprozent zu erreichen.

Die aus dem Verfahren resultierenden Kristalle zeigen rasterelektronenmikroskopisch stellenweise eine leichte Trübung an der Oberfläche durch die Bildung von Citrat-Ionen.

Da es sich erfindungsgemäß vornehmlich um die Herstellung passivierter Zitronensäure für den Einsatz zur Herstellung von Brausetabletten handelt, ist es nötig, den Gehalt an reaktionsfähiger Zitronensäure so hoch wie möglich zu halten, gleichzeitig aber den Gehalt an Alkali- und/oder Erdalkali-Dotierung so einzustellen, daß einerseits beim Auflösen in Wasser ein genügend starker Brauseeffekt mit den CO₂-abspaltenden Bestandteilen der Brausemischung erzeugt werden kann, andererseits aber eine genügende Passivierung der Zitronensäure vorliegt, damit bei einer Mischung mit Erdalkali- bzw. Alkali(hydrogen)carbonaten bzw. anderen CO₂abspaltenden Bestandteilen die gewünschte verminderte Reaktivität zwischen Säurekristallen und CO₂-abspaltenden Bestandteilen vor dem bestimmungsgemässen Gebrauch der Brausemischung bzw. Brausetablette, d.h. während der Lagerung, zum Tragen kommt. Es wird auch angestrebt, eine möglichst gleichmäßige Dotierung und Passivierung der Oberfläche der Zitronensäure zu erhalten.

Die Empfindlichkeit von Tabletten gegenüber Feuchtigkeit wird in der Feuchten Kammer untersucht. Die Feuchte Kammer ist ein geschlossenes System, eingestellt auf 80% relative Feuchte. Die Tabletten werden in diese Feuchte Kammer eingebracht; die Veränderung der Tablette wird visuell bzw. mittels Time Lapse Kamera beobachtet. Dann kann man Veränderungen an der Oberfläche der Tablette durch beginnende Reaktion feststellen, wie auch die Gewichtszunahme oder -abnahme und die Veränderung der Auflöszeit messen.

Die erfindungsgemäß hergestellte, oberflächendotierte organische Säure, insbesondere Zitronensäure, zeichnet sich nicht nur durch eine verbesserte Verpreßbarkeit sowie durch eine geringere Reaktivität gegenüber Erdalkali- bzw. Alkali(hydrogen)carbonaten aus sondern zeigt auch eine wesentlich verbesserte Stabilität bei Feuchtigkeitseinfluß.

Die Erfindung wird im folgenden anhand einiger Beispiele näher erläutert:

### Beispiel 1:

### Behandlung der Zitronensäure mit Trinatriumcitrat-Lösung, entsprechend einer Natriumdotierung von 1,29 Gew.% (=Gewichtsprozent), bezogen auf die Zitronensäure:

1600 Gew.teile (= Gewichtsteile) feinkristalline Zitronensäure werden auf 60°C aufgeheizt und stufenweise mit einer Lösung von 80 Gew.teilen Trinatriumcitrat in 88 Gew.teilen Wasser versetzt. Die Zitronensäure wird anschließend getrocknet und auf eine maximale Korngrösse von 0,5 mm gesiebt. Für Preß- und Stabilitätsversuche wurden 2457 Gew.teile dotierter Zitronensäure mit 1296 Gew.teilen Natriumhydrogencarbonat sowie 179,2 Gew.teilen Natriumcarbonat vermischt und zu Tabletten verpreßt. Die Tabletten zeigten - verpreßt auf einen Stempeldurchmesser von 22 mm - eine maximal mögliche Härte von 13,5 kp, eine Auflöszeit von 55 Sekunden sowie eine wesentlich bessere, zweieinhalbfache Stabilität in der Feuchten Kammer gegenüber unbehandelter Zitronensäure.

### Beispiel 2:

500 Gew.teile feinkristalline Zitronensäure wasserfrei sowie 500 Gew.teile feinkristalline Zitronensäure-Monohydrat werden mit 400 Gew.teilen Trinatriumcitrat x 2H₂O unter Rühren auf 70°C aufgeheizt, wobei das Trinatriumcitrat an der Oberfläche der Zitronensäure anreagiert und an der Oberfläche zumindest Mononatriumcitrat bildet. Nach Erreichen von 70°C wird eine Stunde bei 80°C getrocknet. Die Trocknung kann mittels Vakuum durchgeführt werden oder auch im Trockentunnel. Anschließend wird die Zitronensäure auf die gewünschte Körnung gesiebt. Die so behandelte Zitronensäure kann dann in Mischung mit Natriumhydrogencarbonat zu Brausetabletten verpreßt werden, indem man 2760 Gew.teile der behandelten Zitronensäure mit 540 Gew.teilen Natriumbicarbonat mischt und zu Tabletten verpreßt. Man erhält Härten von 8 - 9 kp und eine Auflöszeit von 40 Sekunden bei einem pH von 4,28. Die Empfindlichkeit der Brausetablette gegenüber Luftfeuchtigkeit ist im Vergleich zu einer unbehandelten Zitronensäure, bei der die Brausetablette bereits nach wenigen Minuten oberflächliche Veränderungen zeigt, signifikant, nämlich auf etwa ein Drittel, vermindert.

### Beispiel 3:

### O,27% Natrium-Ionendotierung auf die Zitronensäure:

1600 Gew.teile feinkristalliner Zitronensäure werden erwärmt; man bringt eine Lösung von 16 Gew.teilen Trinatriumcitrat in 17,6 Gew.teilen Wasser auf die Zitronensäure auf und verteilt gleichmäßig unter Rühren. Nach einer Einwirkzeit von 5 Minuten wird das Produkt 30 min im Vakuum getrocknet und anschließend auf eine Korngrösse von 0,5 mm gesiebt.

Mischung zur Direktverpressung einer Brausebasis: 1670 Gew.teile der wie oben beschriebenen behandelten Zitronensäure werden mit 567 Gew.teilen Natriumhydrogencarbonat sowie 73 Gew.teilen Natriumcarbonat vermischt und zu Tabletten von 3,9 g verpreßt. Man erreicht eine Härte von 9 kp und eine Auflöszeit von 45 Sekunden. Die Feuchtigkeitsstabilität in der Feuchten Kammer ist gegenüber unbehandelter Zitronensäure ca. doppelt so gut.

### Beispiel 4:

### Behandlung einer Zitronensäure mit Natronlauge:

1000 Gew.teile kristalliner Zitronensäure werden mit einer 0,1N-Natronlauge gewaschen, wobei eine Natrium-Dotierung von ca. 0.025% Na (auf Zitronensäure gerechnet) erzielt wird. Die Natronlauge kann aber auch unter Rühren aufgesprüht und gleichmäßig verteilt werden, worauf die so behandelte Zitronensäure - gegebenenfalls kontinuierlich - in einem Heißlufttunnel getrocknet wird. Das so erhaltene Produkt kann in diesem Zustand gelagert oder als Ausgangsmaterial für spezifische Anwendungen weiterverwendet werden.

Für die Direktverpressung von Brausetabletten werden 1670 Gew.teile der wie oben beschriebenen behandelten Zitronensäure mit 567 Gew.teilen Natriumhydrogencarbonat sowie 73 Gew.teilen Natriumcarbonat vermischt. Man erhält eine Härte von 7,5 - 8 kp und eine Auflöszeit von 45 Sekunden. Die Stabilität in der Feuchten Kammer ist gegenüber einer Tablette mit unbehandelter Zitronensäure um 50% verbessert.

### Beispiel 5:

Die Zitronensäure kann aber auch mit 0,1N Natronlauge in Ethanol mit Natriumionen dotiert werden, wobei 1000 Gew.teile Zitronensäure mit 0,1 N-Natronlauge in Ethanol gewaschen und anschließend getrocknet werden. Die Natriumdotierung beträgt 0,1 Gew.% Natrium auf die Zitronensäure. Eine Mischung der so behandelten Zitronensäure von 1680 Gew.teilen mit 886 Gew.teilen Natriumhydrogencarbonat und 123 Gew.teilen Natriumcarbonat, verpreßt auf ein Tablettengewicht von 2680 mg ergibt eine Härte von 10 - 11 kp, einen pH-Wert von 4,55 und eine Auflöszeit von 40 Sekunden. Die Stabilität in der Feuchten Kammer zeigt auch in diesem Fall eine wesentliche Verbesserung, nämlich auf das eineinhalbfache, gegenüber Tabletten auf der Basis unbehandelter Zitronensäure.

Mittels dispersiver Röntgenspektroskopie konnten an den erfindungsgemässen Zitronensäurekristallen bis zu 56 Atomprozent Na festgestellt werden.

## Patentansprüche

1. Oberflächenbehandelte Kristalle einer festen, essbaren organischen Säure, **dadurch gekennzeichnet, daß** ein Teil der ursprünglichen Säuremoleküle in der Oberfläche der Kristalle in Form von carbonatfreien Ammonium-, Alkali- oder Erdalkalisalzen der Säure, vorliegt, wobei die Oberfläche der Säurekristalle einen Anteil von 0,01-1,5 Gew.%, bezogen auf die organische Säure, an Ammonium-, Alkali- und/oder Erdatkali-tonen enthält.

2. Kristalle nach Anspruch 1, wobei die organische Säure eine Zitronensäure ist und ein Teil der ursprünglichen Säuremoleküle in der Oberfläche der Kristalle in Form von Ammonium-, Alkali- oder Erdalkalicitrat vorliegt.

3. Säurekristalle nach einem der Ansprüche 1 oder 2,wobei die Kristalloberfläche zumindest eines der nachfolgenden Elemente: Na, K, Ca, in Form eines Salzes der Säure enthält.

4. Verfahren zur Herstellung von Säurekristallen nach Anspruch 1, **dadurch gekennzeichnet, dass** Kristalle einer essbaren organischen Säure an der Oberfläche mittels Alkali- und/oder Erdalkalisalzen oder einer Alkali- und/oder Erdalkali-Ionen enthaltenden Lösung oder mittels Ammoniakdampf behandelt werden, wobei durch chemische Reaktion in der Kristalloberfläche Moleküle der essbaren organischen Säure in Ammonium-, Alkali- und/oder Erdalkalisalze umgewandelt werden, und dass nach einer vorbestimmten Reaktionsdauer die Reaktion gestoppt und die behandelten Säurekristalle getrocknet werden.

5. Verfahren nach Anspruch 4, wobei Kristalle der Zitronensäure behandeldt werden, die durch chemische Reaktion in der Kristalloberfläche in Ammonium-, Alkali- und/oder Erdalkaticitrate umgewandelt werden.

6. Verfahren nach einem der Ansprüche 4 oder 5, wobei als Alkali- und/oder Erdalkali-Salze oder zur Herstellung von Alkali- und/oder Erdalkali-Ionen enthaltenden Lösungen Citrate und/oder Hydroxyde der jeweiligen Elemente, insbesondere von Natrium-, Kalium- oder Calcium, eingesetzt werden.

7. Verfahren nach einem der Ansprüche 4 bis 7, wobei die Oberflächenbehandlung bei einer gegenüber Raumtemperatur erhöhten Temperatur, vorzugsweise bei 60 bis 70°C erfolgt.

8. Säurekristalle nach Anspruch 1, herstellbar nach einem der Ansprüche 4 bis 8.

9. Säurekristalle nach einem der Ansprüche 1, 2, 3 oder 9 zur Verwendung im Getränke- und Nahrungsmittelbereich oder zur Herstellung von chemischen und/oder pharmazeutischen Produkten, insbesondere zur Herstellung von Brauseformulierungen.

## Claims

1. Surface-treated crystal of a solid, edible organic acid, **characterized in that** some of the original acid molecules in the surface of the crystal are present in the form of carbonate-free ammonium, alkali metal or alkaline earth metal salts of the acid, the surface of the acid crystal containing a proportion of 0.01-1.5% by weight, based on the organic acid, of ammonium, alkali metal and/or alkaline earth metal ions.

2. Crystals according to Claim 1, the organic acid being citric acid and some of the original acid molecules in the surface of the crystals being present in the form of ammonium, alkali metal or alkaline earth. metal citrate.

3. Acid crystals according to either of Claims 1 and 2, the crystal surface containing at least one of the following elements: Na, K, Ca, in the form of a salt of the acid.

4. Process for the preparation of acid crystals according to Claim 1, **characterized in that** crystals of an edible organic acid are treated on the surface by means of alkali metal and/or alkaline earth metal salts or a solution containing alkali metal and/or alkaline earth metal ions or by means of ammonia vapour, molecules of the edible organic acid being converted into ammonium, alkali metal and/or alkaline earth metal salts by chemical reaction in the crystal surface, and that, after a predetermined reaction time, the reaction is stopped and the treated acid crystals are dried.

5. Process according to Claim 4, crystals of citric acid being treated, which are converted into ammonium, alkali metal and/or alkaline earth metal citrates by chemical reaction in the crystal surface.

6. Process according to either of Claims 4 and 5, citrates and/or hydroxides of the respective elements, in particular of sodium, potassium or calcium, being used as alkali metal and/or alkaline earth metal salts or for the preparation of solutions containing alkali metal and/or alkaline earth metal ions.

7. Process according to any of Claims 4 to 6, the surface treatment being carried out at a temperature higher than room temperature, preferably at 60 to 70°C.

8. Acid crystals according to Claim 1, which can be prepared according to any of Claims 4 to 7.

9. Acid crystals according to any of Claims 1, 2, 3 and 8 for use in the beverage and food sector or for the preparation of chemical and/or pharmaceutical products, in particular for the preparation of effervescent formulations.

## Revendications

1. Cristaux d'un acide organique solide de qualité alimentaire ayant subi un traitement de surface, **caractérisés en ce qu'**une partie des molécules de l'acide de départ de la surface des cristaux sont présentes sous la forme - exempte de carbonate - d'un sel d'ammonium, de métal alcalin ou de métal alcalino-terreux de cet acide, la surface des cristaux de l'acide contenant une quantité correspondant à 0,01 - 1,5 % en poids par rapport à l'acide organique, d'ions ammonium, d'ions de métal alcalin et / ou d'ions de métal alcalino-terreux.

2. Cristaux selon la revendication 1, dans lesquels l'acide organique est un acide citrique et une partie des molécules de l'acide de départ à la surface des cristaux sont sous la forme de citrate d'ammonium, de métal alcalin ou de métal alcalino-terreux.

3. Cristaux selon la revendication 1 ou la revendication 2, dans lesquels la surface des cristaux contient au moins un des éléments suivants, sous la forme d'un sel de l'acide : Na, K, Ca.

4. Procédé pour produire des cristaux d'acide selon la revendication 1, **caractérisé en ce que** les cristaux d'un acide organique de qualité alimentaire sont traités en surface par des sels de métal alcalin et/ou alcalino-terreux, ou par une solution contenant des ions de métal alcalin et / ou des ions de métal alcalino-terreux ou encore par des vapeurs d'ammoniac, grâce à quoi des molécules de l'acide organique de qualité alimentaire sont converties au niveau de la surface des cristaux, par réaction chimique, en sel d'ammonium, de métal alcalin et / ou de métal alcalino-terreux et **en ce qu'**après une durée de réaction prédéterminée, cette réaction est interrompue et les cristaux d'acide traités sont séchés.

5. Procédé selon la revendication 4, dans lequel les cristaux de l'acide citrique sont traités par une réaction chimique, dans laquelle la surface des cristaux est convertie en citrate d'ammonium, de métal alcalin et / ou de métal alcalino-terreux.

6. Procédé selon la revendication 4 ou la revendication 5, dans lequel on utilise, en tant que sel de métal alcalin et / ou alcalino-terreux ou pour préparer les solutions contenant des ions de métal alcalin et / ou alcalino-terreux, le citrate et / ou l'hydroxyde de l'élément considéré, en particulier de sodium, de potassium ou de calcium.

7. Procédé selon l'une des revendications 4 à 7, dans lequel le traitement de la surface se fait à une température augmentée par rapport à la température ambiante et, de préférence, à 60 - 70 °C.

8. Cristaux d'acide selon la revendication 1, préparés selon l'une des revendications 4 à 8.

9. Cristaux d'acide selon l'une des revendications 1, 2, 3 ou 9, pour une utilisation dans le secteur des boissons et des aliments ou pour la préparation de produits chimiques et / ou pharmaceutiques et, en particulier, pour la préparation de formules effervescentes.
